# DEMANDE DE BREVET EUROPEEN

(11) **EP 2 772 499 A1**
(43) Date de publication de la demande: **03.09.2014**
(21) Numéro de dépôt: 14166826.9
(22) Date de dépôt: 29.06.2006
(51) Int. Cl.: C07K 14/745, C07K 14/75, C07K 14/78, C12N 9/10

(54) **Separation de proteines plasmatiques**

(30) Priorité: 29.06.2005 FR 0506640
(62) Demande divisionnaire de: 06013497.0
(71) Demandeur: Laboratoire Français du Fractionnement et des Biotechnologies, 91940 Les Ulis (FR)
(72) Inventeur: Nogre, Michel, 92170 VANVES (FR); Porte, Pierre, 91720 PRUNAY-SUR-ESSONNE (FR); Tellier, Michel, 95600 EAUBONNE (FR)
(74) Mandataire: Hirsch & Associés

(57) **Abrégé**

L'invention concerne un procédé de separation des protéines fibrinogène, Facteur XIII et colle biologique d'une fraction plasmatique solubilisée et de préparation de concentrés lyophilisés desdites protéines comprenant les étapes de :
- purification chromatographique comprenant les étapes dé chargement d'un échangeur d'anions de type base faible en ladite fraction solubilisée, préalablement équilibré par un tampon de force ionique prédéterminée de pH basique, ce qui permet la rétention de la colle biologique, élution de la colle biologique par augmentation de la force ionique dudit tampon, et
- séparation du FXIII à partir du fibrinogène par ajout dans au moins une partie de l'éluat de colle biologique d'au moins un agent chimique précipitant du FXIII, et récupération de la solution résultante de surnageant de fibrinogène purifié. Elle concerne également les concentrés lyophilisés desdites protéines susceptibles d'être obtenus par la mise en oeuvre du procédé.

## Description

La présente invention concerne un procédé de séparation des protéines fibrinogène, Facteur XIII et colle biologique et de préparation de concentrés lyophilisés desdites protéines, hautement purifiés et elle concerne également lesdits concentrés lyophilisés susceptibles d'être obtenus par le procédé.

Le fibrinogène est une protéine essentielle de la coagulation sanguine, car sa polymérisation en fibrine insoluble formée au terme de la cascade des réactions qui gouvernent la coagulation, aboutit à la formation d'un caillot obturant la brèche vasculaire, responsable du saignement. La mise en place du caillot est ainsi essentielle pour assurer l'arrêt du saignement. De plus, la fibrine formée au niveau de la plaie constitue un réseau fibrillaire qui assure la réparation tissulaire (cicatrisation).

Des déficiences congénitales en fibrinogène peuvent conduire à de graves pathologies. Pour soigner ces déficiences, il est nécessaire de disposer de concentrés de fibrinogène pouvant être administrés à des patients en traitement. D'autres pathologies peuvent également être soignées par des apports de fibrinogène, notamment en cas de pertes massives de sang (chirurgie, traumatismes etc.), ou à la suite d'une coagulopathie de consommation décompensée (CIVD).

Par ailleurs, les colles biologiques activables par la thrombine renfermant du fibrinogène, en tant que constituant majeur, et du Facteur XIII (FXIII), sont efficacement utilisées pour la réparation tissulaire lors d'usages cliniques, tels les greffes de peau, les sutures nerveuses ou artérielles, comme décrit, par exemple, dans les brevets EP 0 305 243, FR 2 448 900 et FR 2 448 901. La présence de Facteur XIII ou transglutaminase dans ces produits contribue à stabiliser la fibrine par la création de liaisons covalentes intercaténaires qui la rendent insoluble. Dans certains cas, ces produits sont obtenus selon des procédés de production du fibrinogène assez complexes qui nécessitent un apport exogène de Facteur XIII purifié afin qu'ils puissent remplir leur fonction thérapeutique.

Par conséquent, la mise à disposition de concentrés de fibrinogène, de colles biologiques, et de Facteur XIII, notamment à des fins thérapeutiques, requiert des techniques de purification conduisant à ces produits non seulement suffisamment purifiés de contaminants de nature diverse; tels que les protéines accompagnantes ou coprécipitées, les anticorps ou" les protéases, mais de surcroît sécurisés sur le plan viral.

L'isolement de fractions enrichies en fibrinogène, contenant éventuellement du FXIII, à partir du plasma est connu et a été décrit initialement par les travaux de Cohn et Nitschmann (Cohn et al, J. Am. Chem. Soc., 68, 459, 1946 et Kistler et al, Vox Sang., 7, 1962, 414-424). Des méthodes plus récentes associent des techniques de précipitation de différentes sources de plasma et des techniques de filtration, de chromatographie, d'inactivation virale etc. On peut citer, à titre d'exemple, les brevets et demandes de brevets EP 0 359 593, US 5 099 003, EP 0 305 243, FR 2 448 900 et FR 2 448 901.

Toutefois, des procédés différents sont à chaque fois mis en oeuvre pour la production de concentrés ou de compositions soit de fibrinogène, comme décrit dans la demande de brevet EP 1 457 497 , soit de colle biologique, par exemple selon le brevet EP 0 771 324, ou bien encore enrichis en fibrinogène contenant d'autres protéines accompagnantes comme le FXIII, le Facteur VIII, la fibronectine, le facteur von Willebrand etc. (notamment US 6 121 232).

Ces procédés impliquent donc des chaînes de production distinctes utilisant, par conséquent, des méthodes différentes pour l'obtention des protéines considérées avec l'emploi de plusieurs sources de matière première à chaque fois. En outre, elles peuvent impliquer, selon les cas, des supports chromatographiques coûteux, tels que des gels d'affinité à base de métal chelaté (WO 2004/007533), susceptibles toutefois de relarguer des métaux résiduels dans les éluats, lesquels peuvent entraîner des réactions indésirables avec les protéines (oxydation par exemple). Ceci pose des problèmes de lourdeur de mise en oeuvre à l'échelle industrielle lorsque des besoins conjoints en ces trois, principes actifs purifiés se font ressentir. Ces problèmes sont encore accentués, lorsque les différentes protéines ainsi obtenues doivent subir des traitements pour rendre inactifs et/ou éliminer les virus et autres contaminants indésirables, comme le prion.

A cette fin, certains traitements d'inactivation virale classiques consistant en un traitement thermique, tel que la pasteurisation à 60°C pendant 20 h en présence de stabilisants protecteurs, et un traitement chimique, tel que par solvant-détergent, destinés à rendre des concentrés cités ci-dessus compatible à un usage thérapeutique, ne permettent pas une complète inactivation des virus, notamment des virus non enveloppés (parvovirus B19, hépatite A et B etc.).

Pour remédier à cet inconvénient, on recourt habituellement à des méthodes d'inactivation virales plus efficaces, telles qu'un chauffage à sec en conditions sévères (80°C, 72 h). Cette étape nécessite l'incorporation d'une formulation stabilisante adéquate offrant des conditions telles que, par exemple, le fibrinogène soit stabilisé durant cette étape, alors que les virus sont détruits. Une telle formulation a fait l'objet d'une demande de brevet FR 04 02001 déposée par la Demanderesse. Or, cette formulation s'applique à la stabilisation d'une protéine définie et non des protéines accompagnantes, dont les caractéristiques sont différentes du fibrinogène.

Les techniques de filtration, en particulier la nanofiltration utilisant des filtres de porosité de 35 nm, voire inférieure, ont également été mises en oeuvre pour l'élimination virale. Cependant, cette technique ne peut être efficacement utilisée sans la maîtrise des paramètres physico-chimiques influant sur le rendement de récupération des composés à filtrer, et ce en évitant le colmatage du filtre et le passage de divers virus et contaminants. Ces paramètres, tels que la force ionique, le pH de la solution, ainsi que les conditions opératoires de la filtration, imposent des conditions spécifiques de mise en oeuvre qui dépendent également de la nature du ou des composés présents dans la solution à filtrer. Bien que les demandes de brevet EP 1 348 445 Al, EP 1 161 958 A1 et WO 99/23111 divulguent l'élimination quasi-totale des virus non enveloppés de très petite taille présents dans des solutions protéiniques, tels que ceux de l'hépatite A, par une nanofiltration avec des filtres de 15 nm, il demeure néanmoins toujours un risque de transmission de virus indésirables ou de prions.

Pour remédier à ce risque, on peut mettre en oeuvre une double, voire triple, inactivation et/ou élimination virale associant au moins deux quelconques des techniques mentionnées ci-dessus, comme décrit par exemple dans la demande de brevet WO 2004/007533. Lorsqu'on associe de tels traitements, il devient alors indispensable de choisir, en fonction de la méthode d'inactivation virale, des excipients virucides et/ou des stabilisants protecteurs n'ayant pas simultanément une action néfaste par exemple sur les paramètres physico-chimiques cités ci-dessus régissant la nanofiltration.

La Demanderesse a donc cherché à mettre au point un procédé de séparation de fibrinogène, de Facteur XIII et de colle biologique activable par la thrombine répondant à un double objectif. D'une part, mettre à disposition un procédé unique permettant l'obtention conjointe de concentrés de ces protéines lyophilisées et hautement purifiées, à partir d'une seule matière première plasmatique contenant du fibrinogène et du Facteur XIII, et, d'autre part que ce procédé soit compatible avec au moins un traitement d'inactivation et/ou d'élimination des virus et d'autres contaminants indésirables (polymères, agrégats, prion).

L'invention concerne, par conséquent, un procédé de séparation des protéines fibrinogène, Facteur XIII et colle biologique d'une fraction plasmatique solubilisée, à base de fibrinogène et de Facteur XIII, et de préparation de concentrés lyophilisés desdites protéines comprenant les étapes de :
a) purification chromatographique comprenant les étapes de :
   i)chargement d'un échangeur d'anions de type base faible en ladite fraction solubilisée, préalablement équilibré par un tampon de force ionique prédéterminée de pH basique, ce qui permet la rétention de la colle biologique,
   ii) élution de la colle biologique par augmentation de la force ionique dudit tampon,
b) séparation du FXIII à partir du fibrinogène par ajout dans au moins une partie de l'éluat de colle biologique d'au moins un agent chimique précipitant du FXIII, et récupération de la solution résultante de surnageant de fibrinogène purifié, et
c) diafiltration des solutions de fibrinogène, de colle biologique et de FXIII remis en solution, suivie d'une lyophilisation desdites solutions.

Ainsi, la Demanderesse a trouvé qu'il était possible d'obtenir, à l'échelle industrielle, des concentrés de fibrinogène, de Facteur XIII et de colle biologique lyophilisés, hautement purifiés, dépourvus de protéines coprécipitées et de contaminants indésirables, par la mise en oeuvre d'un seul procédé flexible qui, en fonction des besoins, permet d'ajuster au mieux la production en chacun des composés considérés, tout en assurant une rentabilisation optimum de la matière première. Un tel procédé simple, rapide et peu coûteux est aisément mis en oeuvre à l'échelle industrielle, ce qui conduit à une optimisation accrue des divers schémas de production.

En outre, en fonction de la matière première utilisée et des utilisations envisagées, l'ajout d'étapes supplémentaires à ce procédé de traitement d'inactivation et/ou d'élimination virale conduit aux trois concentrés d'intérêt, appropriés à des usages thérapeutiques.

Selon l'invention, on peut utiliser plusieurs sources de matière première contenant du fibrinogène et du Facteur XIII. Ainsi, ces fractions plasmatiques sont obtenues par fractionnement de plasma recueilli dans des conditions défavorables au maintien d'un taux suffisant de Facteur VIII, qui est une protéine labile, selon la méthode de Cohn utilisant de l'alcool froid. Il est également envisageable d'appliquer le fractionnement précédent à un cryoprécipité, remis préalablement en solution, dès lors que l'extraction du Facteur VIII n'est pas envisagée, par exemple dans le cas d'un cryoprécipité périmé ou non conforme par rapport à la teneur minimale en Facteur VIII, ou à du plasma dépourvu de cryoprécipité. Ces différentes sources de fibrinogène constituent donc un précipité de fraction I de Cohn, qui, après lavage, est ensuite solubilisé dans tout tampon approprié de pH neutre, connu de l'homme du métier. A titre d'exemple, un tel tampon est à base de chlorure de sodium, de citrate trisodique et L-arginine, de pH voisin de 7,4, dont la concentration en chacun des composants représente respectivement, de façon préférée, 0,12 M, 0,01 M et 0,05 M.

La flexibilité du procédé de l'invention est donc également liée à la variété des matières premières susceptibles de contenir du fibrinogène extractible, dont la purification conduit aux trois concentrés considérés, pour des usages thérapeutiques ciblés.

Le procédé peut également comprendre, avant l'étape a), une étape initiale de prépurification de la fraction plasmatique solubilisée par un traitement à l'hydroxyde d'aluminium et/ou une précipitation à basse température. L'ajout d'hydroxyde d'aluminium assure l'élimination des protéines indésirables, telles que les Facteurs II (FII), VII, IX et X (FX). Cette fraction prépurifiée peut être congelée dans l'attente de la mise en oeuvre d'étapes ultérieures du procédé selon l'invention.

La purification chromatographique de la fraction plasmatique solubilisée est effectuée sur toute matrice à base de polymère naturel ou synthétique, résine ou gel, sur laquelle sont greffés des groupements échangeurs d'anions de type base faible, tel que le DEAE. Les supports chromatographiques classiques de ce type sont disponibles sous les appellations DFAE-Sepharose^{®} CL-6B, DEAS-Trisacryl LS, Fractogel TSK-DEAE 650 M ou S, DEAE-Macroprep (Bio-Rad, France) etc.

Le tampon d'équilibrage de l'échangeur d'anions présente une force ionique prédéterminée et doit être à un pH basique. La force ionique est typiquement inférieure à la valeur de 0,2 et, de préférence, est située dans la plage de valeurs allant de 0,06 à 0,2, en particulier, de 0,08 à 0,15. Celle-ci est de préférence ajustée par ajout de sels inorganiques de métaux alcalins ou alcalino-terreux ou leur mélange, de façon très préférée de sels inorganiques de métaux alcalins, en particulier de chlorure de sodium.

La valeur maximale du pH du tampon d'équilibrage est choisie de façon à éviter toute dénaturation des produits considérés, à savoir environ 10. Avantageusement, le pH est situé dans la plage de valeurs supérieures à 7 jusqu'à 9, de préférence de 7,5 à 8,2. A titre d'exemple, ce tampon est composé de chlorure de sodium, à un pH de 7,9-8,1, dont la concentration est de 0,06 M, et peut très préférentiellement comprendre en plus du citrate trisodique, à une concentration préférée de 0,011 M. On peut également utiliser tout autre tampon, à base de chlorure de sodium ou de sels inorganiques de métaux alcalins ou alcalino-terreux, comprenant d'autres composés biologiquement compatibles et non dénaturants pour les produits d'intérêt.

Quand la solution ci-dessus a été appliquée sur l'échangeur d'anions, la colle biologique est retenue sur le support. Le procédé peut comprendre, préalablement à l'étape d'élution de la colle biologique, une étape de lavage, avec ledit tampon d'équilibrage, de l'échangeur d'anions jusqu'à l'élimination des protéines, et des contaminants non retenus. Cette étape de lavage permet, par percolation de ce tampon sur le support, le passage dans le filtrat de protéines présentes dans la solution contenant du fibrinogène, telles les immunoglobulines G (IgG), A (IgA) et M (IgM) et l'albumine, et des contaminants non retenus ou faiblement retenus par l'échangeur, comme les agents chimiques d'inactivation virale. La durée du lavage est déterminée par mesure de la densité optique (DO) du filtrat à la longueur d'onde de 280 nm. En effet, une valeur de DO correspondant à celle de la ligne de base est une bonne indication de l'élimination effective des composés cités ci-dessus.

Après retour à la ligne de base, l'élution de la colle biologique est effectuée par augmentation de la force ionique du tampon d'équilibrage ou de lavage, dont le pH est de préférence fixé à 7,4-7,6. La valeur de cette force ionique est choisie de façon à obtenir l'élution efficace de la colle biologique tout en veillant à ce que cette valeur n'altère pas les propriétés du produit considéré. Avantageusement, la valeur de la force ionique est comprise entre 0,5 et 1,3, en particulier entre 0,9 et 1,1. Cette augmentation de la force ionique est effectuée par ajout de tout sel ou mélange de sels définis ci-dessus, notamment de chlorure de sodium. Le tampon d'élution peut en outre contenir d'autres excipients, tel qu'un mélange de constituants, dénommé mélange A, comprenant le citrate trisodique (10 à 12 g/l), la lysine (1 à 5 g/l), la glycine (1 à 5 g/l), le sel tris (2 à 5 g/l), l'arginine (25 à 50 g/l) et l'isoleucine (5 à 15 g/l). La concentration en protéines dans l'éluat est de l'ordre de 4 g/l.

Au moins une partie de la quantité récoltée de l'éluat de colle biologique est soumise à un traitement destiné à séparer le FXIII accompagnant le fibrinogène. Cette séparation est réalisée en faisant précipiter le FXIII par ajout dans l'éluat à traiter d'un agent chimique précipitant, pouvant être sous la forme d'une solution aqueuse, à une concentration permettant d'obtenir l'effet voulu. La préférence étant donnée à des solutions aqueuses à base de sels de citrate 1 M, tels que le citrate de sodium et, en particulier, le citrate trisodique. On sépare ainsi un précipité de FXIII et un surnageant très enrichi en fibrinogène. Une récupération très efficace du précipité de FXIII peut être obtenue par filtration sur des filtres de 5 µm.

Le précipité de FXIII ainsi récupéré est remis en solution, de préférence dans de l'eau ou un tampon. En particulier, il est dissout dans un tampon de mélange A, ajusté à un pH de 6,9-7,1, pour que sa concentration corresponde à une activité environ 100 fois supérieure à celle du plasma normal. A ce titre, le précipité de FXIII peut par exemple être repris de façon que sa concentration soit d'environ 1 à 5 g de protéines totales/l.

Selon l'invention, les solutions de colle biologique (éluat de colle biologique) et de fibrinogène (surnageant enrichi en fibrinogène) peuvent être concentrées par ultrafiltration, jusqu'à des teneurs typiquement comprises entre 15 et 25 g de protéines totales/l déterminées par des mesures classiques connues de l'homme du métier.

Les trois solutions de fibrinogène, de Facteur XIII et de colle biologique obtenues, éventuellement concentrées, sont soumises à une étape de diafiltration. Cette étape est tout d'abord destinée à éliminer l'excès éventuel, d'une, part, de sel inorganique utilisé pour obtenir des solutions ayant une force ionique d'au plus 0,2 M, et, d'autre part, d'agent précipitant présent dans le précipité remis en solutions. Il est à noter qu'une présence importante de sel inorganique; nécessaire à l'élution de la colle biologique, aurait une influence néfaste sur l'efficacité du processus de lyophilisation et de l'inactivation virale par chauffage thermique à sec ainsi que sur la capacité de rétention des virus par un nanofiltre approprié. Cette étape peut également se révéler nécessaire afin d'incorporer, le cas échéant, des excipients adéquats, stabilisants et protecteurs, destinés à autoriser, d'une part, un chauffage à sec du fibrinogène, du FXIII et de colle biologique sans risque de dénaturation, et, d'autre part, une solubilisation rapide des lyophilisats, typiquement en 3 à 8 min. Le tampon de diafiltration préféré contient le mélange A, et est à pH 6,9-7,1, par référence à la demande de brevet FR 04 02001 déposée par la Demanderesse.

Il convient de signaler qu'un autre avantage de l'invention est que, selon d'autres modes préférés de mise en oeuvre du procédé, le tampon de diafiltration/comprenant le mélange A peut déjà entrer dans la constitution du tampon d'élution de la colle biologique. La mise en oeuvre de la diafiltration s'en trouve donc simplifiée et optimisée.

Des tampons de diafiltration de composition différente peuvent aussi être utilisés en fonction des besoins, à condition qu'ils remplissent les critères énoncés ci-dessus.

L'étape d'ultrafiltration mentionnée plus haut peut être également mise oeuvre dans les mêmes conditions à ce stade du procédé.

Les solutions respectives éventuellement diafiltrées, concentrées le cas échéant, sont lyophilisées selon des méthodes classiques et conditions habituelles, à savoir entre -40°C et -30°C pendant environ 48 heures.

Il peut en outre être prévu au moins une étape de traitement d'inactivation virale et/ou d'élimination de virus et des contaminants cités précédemment, comme le prison. Ce traitement peut être choisi dans le groupe constitué par le traitement chimique d'inactivation virale, la nanofiltration et le traitement thermique d'inactivation virale à sec.

Ainsi, cette étape peut être effectuée par un traitement chimique classique d'inactivation virale, de préférence consistant en un traitement par solvant-détergent, suivant la méthode décrite dans le brevet EP 0 131 740. Les agents chimiques d'inactivation virale représentent préférentiellement le mélange de Tween^{®}-TnBP, de façon plus préférée le mélange de Triton^{®} (octoxinol)-TnBP, dont les concentrations typiques sont respectivement de 0,3% (v/v) et 1% (p/v). Cette inactivation virale peut être intégrée à un stade quelconque du procédé, mais elle est judicieusement mise en oeuvre avant l'étape a) de purification chramatographique. De cette façon, celle-ci contribuera à l'élimination efficace des agents d'inactivation.

Selon un mode préféré de mise en oeuvre du procédé, il peut également être prévu une étape de nanofiltration pour éliminer les virus, notamment non enveloppés, et autres contaminants exogènes, complétant le traitement chimique d'inactivation virale précédent. On peut efficacement utiliser des filtres de 35 nm, bien que d'autres filtres nanométriques puissent être utilisés dans la mesure où les durées de filtration et l'efficacité de rétention virale sont optimisées. La nanofiltration est effectuée soit sur l'éluat obtenu par l'étape a) soit, le cas échéant, sur les solutions diafiltrées de fibrinogène, de colle biologique et de FXIII remis en solution, avant lyophilisation. Le choix judicieux des paramètres chimiques de la purification chromatographique et de celle de la diafiltration permet une flexibilité de mise en oeuvre de la nanofiltration sans en altérer les performances.

Enfin, le traitement thermique d'inactivation virale à sec est effectué sur les lyophilisats de fibrinogène, de colle biologique et de PXIII obtenus après l'étape de lyophilisation, selon des conditions classiques, à 80°C pendant 72 heures, pour l'inactivation des virus non enveloppés qui n'auraient pas été inactivés et/ou éliminés par au moins l'une des deux étapes d'inactivation virale et/ou d'élimination des virus-précédentes.

Les lyophilisats chauffés à sec peuvent ensuite être reconstitués dans un milieu aqueux compatible avec un usage clinique, de préférence dans de l'eau purifiée pour injection (PPI), et directement injectés par voie intraveineuse.

Le procédé selon l'invention peut en outre comporter au moins une étape de filtration clarifiante, pour l'élimination de particules contaminantes insolubles, et au moins une étape stérilisante, celles-ci étant mises en oeuvre de façon habituelle par l'utilisation de filtres, par exemple, de 0,8 à 0,1 µm. En particulier, celles-ci concerneront la fraction plasmatique solubilisée prépurifiée, l'éluat de colle biologique obtenu par l'étape b) et/ou les solutions diafiltrées des trois composés d'intérêt.

Ainsi, la mise en oeuvre du procédé conduit à des lyophilisats de concentrés de colle biologique et de fibrinogène hautement purifiés, dont la teneur respective en fibrinogène, par rapport à la teneur totale de protéines, est d'environ 90%. De plus, les activités de Facteur XIII dans les concentrés de colle biologique et de fibrinogène sont respectivement d'environ 5 U/ml et d'environ 1,5 U/ml.

Le concentré de Facteur XIII obtenu est dépourvu de protéines contaminantes et présente une activité, selon les besoins, située dans la plage de valeurs allant d'environ 30 U/ml à environ 700 U/ml, de préférence de 100 U/ml à 400 U/ml, obtenue en fonction de la concentration de remise en solution du précipité de FKIII et/ou de celle obtenue après ultrafiltration.

L'invention concerne également des concentrés lyophilisés de fibrinogène, de colle biologique et de Facteur XIII, susceptibles d'être obtenus par la mise en oeuvre du procédé ci-dessus, caractérisés en ce qu'ils comprennent le mélange de constituants du tampon de diafiltration (mélange A). De plus, lesdits concentrés peuvent être de qualité thérapeutique grâce à l'ajout dans le procédé de l'invention d'au moins une étape de traitement d'inactivation virale et/ou d'élimination des virus et de contaminants.

L'exemple qui suit illustre un mode de réalisation de la présente invention sans toutefois en limiter la portée.

### Exemple

On utilise 1200 1 de plasma humain dépourvu de cryoprécipité. Ce plasma est soumis à une précipitation éthanolique par la méthode de Cohn, selon des conditions connues de l'homme du métier, de façon telle que la concentration d'éthanol dans le plasma considéré soit de 8% (v/v) et la température du mélange ainsi obtenu soit de -3°C.

Le surnageant et le précipité ainsi obtenus sont ensuite centrifugés. On obtient 10 kg de précipité, qui constitue la fraction I de Cohn impure.

La fraction I de Cohn impure (10 kg) est remise en suspension et lavée par 300 1 de tampon « Blombach », constitué d'un mélange de glycine -1 M, de citrate trisodique 0,055 M et d'éthanol 6,5% (v/v), à un pH de 6,8.

Après centrifugation, on récupère 8 kg de pâte de précipité purifié (fraction I de Cohn purifiée) qui sont dissous, à une température de 37°C, dans 60 1 de tampon constitué d'un mélange de chlorure de sodium 0,12 M, de citrate trisodique 0,010 M et d'arginine 0,05 M, de pH 7,4.

La solution de précipité ainsi obtenue est ensuite soumise à une prépurification par traitement au gel d'alumine, à raison de 108 g pour 1 kg de pâte de précipité, à une température de 25°C et à un pH de 6,9-7,1.

Une fois le traitement de prépurification effectué, la solution est soumise à des filtrations lenticulaires clarifiantes, par l'intermédiaire de filtres en fibres de cellulose (Seitz, type K700) de 0,65 µm et stérilisantes par des filtres de 0,2 µm.

Cette solution prépurifiée est soumise à un premier traitement d'inactivation virale par solvant-détergent en présence de Tween^{®}-TnBP, de concentrations respectives de 0,3% (v/v) et 1% (p/v), selon la méthode décrite dans EP 0 131 740.

La solution prépurifiée ainsi traitée est diluée de 50% par ajout du volume nécessaire d'une solution aqueuse de citrate trisodique 0,010 M.

La solution prépurifiée ainsi obtenue, outre le fibrinogène et le Facteur XIII l'accompagnant, contient encore des protéines indésirables telles que les immunoglobulines G et l'albumine, et des contaminants tels que le Tween^{®}-TnBP. Leur élimination effective est effectuée par la mise en oeuvre d'une étape chromatographique.

A cette fin, la solution prépurifiée est injectée sur une colonne chromatographique remplie d'un gel échangeur d'anions DEAE Macroprep (Bio-Rad, France), préalablement équilibrée en tampon constitué de chlorure de sodium 0,06 M et de citrate trisodique 0,011 M, ajusté à un pH de 8,0 M, d'osmolarité de 130-150 mosmolkg⁻¹. Dans ces conditions, le fibrinogène et le Facteur XIII, constituant la colle biologique, sont retenus par le support. Les protéines faiblement ou non retenues par le support sont éliminées dans le filtrat, ainsi que les Tween^{®} et TnBP, par plusieurs lavages successifs avec le même tampon.

Lorsque la DO, mesurée à 280 nm, est retombée à la valeur de celle de la ligne de base, l'élution du fibrinogène et du Facteur XIII, constituant la colle biologique, est effectuée par un tampon d'élution contenant du chlorure de sodium 1 M et un mélange A' constitué de citrate trisodique (11,2 g/l), de lysine (2,0 g/l), de glycine (2,0 g/l), le sel tris (2,40 g/l), d'arginine (40 g/l) et l'isoleucine (10 g/l), ajusté à un pH de 7,5, d'osmolarité > 2000 mosmolkg⁻¹.

L'éluat de colle biologique purifiée ainsi récupéré est soumis à une nanofiltration sur des filtres PLANOVA (Asahi - Japon) de 35 nm et de 1m² de surface, afin d'éliminer les virus qui n'auraient pas été inactivés par le traitement solvant-détergent ci-dessus. La teneur en protéines totales à ce stade du procédé est d'environ 4,0 g/l de solution.

On isole 50% du volume de l'éluat de colle biologique et une solution de citrate trisodique 1 M est ajoutée au restant du volume d'éluat pour faire précipiter le Facteur XIII. Après s'être assuré que tout le Facteur XIII a précipité, celui-ci est isolé et récupéré par filtration sur des filtres Sartopure (Sartorius - France) de 5 µm.

Le précipité de FXIII ainsi récupéré est remis en solution dans de l'eau purifiée pour injection, à raison d'environ 1 g/l.

Les solutions de colle biologique (éluat) et de fibrinogène, sont concentrées par ultrafiltration sur des filtres Biomax Millipore 100 kDa de 5 m² de surface; de façon à ce que la teneur en protéines de chacune des solutions atteigne 15 g/l.

Les solutions concentrées ci-dessus et la solution de FXIII sont soumises à une diafiltration sur les mêmes filtres que ceux utilisés pour l'ultrafiltration contre le mélange A' défini ci-dessus, de pH 6,9-7,1, d'osmolarité de 590-610 mosmolkg⁻¹, ce qui permet également l'élimination du chlorure de sodium.

Après avoir procédé à une filtration stérilisante sur filtres de 0,45-0,2 µm, on prélève 100 ml des solutions respectives diafiltrées, et on les place dans des flacons en verre en vue d'une lyophilisation mise en oeuvre entre -40°C et -30°C pendant environ 48 heures.

Les lyophilisats de fibrinogène, de colle biologique et de Facteur XIII obtenus sont soumis à une ultime étape d'inactivation virale par chauffage à sec à 80°C pendant 72 heures et stockés en vue d'une utilisation thérapeutique ultérieure.

Les résultats des contrôles de qualité effectués sur 3 lots consécutifs sont indiqués dans le tableau qui suit.

## Revendications

1. Procédé de séparation des protéines fibrinogène, Facteur XIII et colle biologique d'une fraction plasmatique solubilisée, à base de fibrinogène et de Facteur XIII, et de préparation de concentrés lyophilisés desdites protéines comprenant les étapes de :
a) purification chromatographique comprenant les étapes de :
i) chargement d'un échangeur d'anions de type base faible en ladite fraction solubilisée, préalablement équilibré par un tampon de force ionique prédéterminée de pH basique, ce qui permet la rétention de la colle biologique,
ii) élution de la colle biologique par augmentation de la force ionique dudit tampon,
b) séparation du FXIII à partir du fibrinogène par ajout dans au moins une partie de l'éluat de colle biologique d'au moins un agent chimique précipitant du FXIII, et récupération de la solution résultante de surnageant de fibrinogène purifié, et
c) diafiltration des solutions de fibrinogène, de colle biologique et de FXIII remis en solution, suivie d'une lyophilisation desdites solutions.

2. Procédé selon la revendication 1, **caractérisé en ce que** le tampon d'équilibrage a une force ionique inférieure à la valeur de 0,2 et, de préférence, est située dans la plage de valeurs allant de 0,06 à 0,2.

3. Procédé selon la revendication 1 ou 2, **caractérisé en ce que** le pH du tampon d'équilibrage est situé dans la plage de valeurs supérieures à 7 jusqu'à 9, de préférence de 7,5 à 8,2.

4. Procédé selon l'une quelconque des revendications 1 à 3, **caractérisé en ce qu'**il comprend, préalablement à l'étape d'élution de la colle biologique, une étape de lavage, avec ledit tampon d'équilibrage, de l'échangeur d'anions jusqu'à l'élimination des protéines et des contaminants non retenus.

5. Procédé selon l'une quelconque des revendications 1 à 4, **caractérisé en ce que** l'élution de la colle biologique est effectuée par le tampon d'équilibrage dont la force ionique est comprise entre 0,5 et 1,3, en particulier entre 0,9 et 1,1, et est à un pH compris entre 7,4 et 7,6.

6. Procédé selon la revendication 5, **caractérisé en ce que** le tampon d'élution contient en outre un mélange de 10 à 12 g/l de citrate trisodique, de 1 à 5 g/l de lysine, de 1 à 5 g/l de glycine, de 2 à 5 g/l de Tris, de 25 à 50 g/l d'arginine et 5 à 15 g/l d'isoleucine.

7. Procédé selon l'une quelconque des revendications 1 à 6, **caractérisé en ce que** l'agent chimique précipitant du Facteur XIII est sous la forme d'une solution aqueuse à base de sels de citrate 1 M.

8. Procédé selon l'une quelconque des revendications 1 à 7, **caractérisé en ce que** le précipité de FXIII est remis en solution dans de l'eau ou dans un tampon contenant un mélange de 10 à 12 g/l de citrate trisodique, de 1 à 5 g/l de lysine, de 1 à 5 g/l de glycine, de 2 à 5 g/l de Tris, de 25 à 50 g/l d'arginine et 5 à 15 g/l d'isoleucine, ajusté à un pH compris entre 6,9 et 7,1.

9. Procédé selon l'une quelconque des revendications 1 à 8, **caractérisé en ce que** la diafiltration est effectuée contre le tampon défini à la revendication 8.

10. Procédé selon l'une quelconque des revendications 1 à 9, **caractérisé en ce qu'**il comprend au moins une étape de traitement d'inactivation virale et/ou d'élimination de virus et de contaminants, le traitement étant choisi dans le groupe constitué par le traitement chimique d'inactivation virale, la nanofiltration et le traitement thermique d'inactivation virale à sec.

11. Procédé selon la revendication 10, **caractérisé en ce que** le traitement chimique d'inactivation virale est effectué préalablement à l'étape a).

12. Procédé selon la revendication 10 ou 11, dans lequel le traitement chimique d'inactivation virale consiste en un traitement par solvant-détergent effectué par des agents chimiques représentant le mélange de Tween^{®}-TnBP ou de Triton^{®}-TnBP.

13. Procédé selon l'une quelconque des revendications 10 à 12, **caractérisé en ce que** la nanofiltration est effectuée sur l'ëluat obtenu par l'étape a) ou sur les solutions diafiltrées de fibrinogène, de colle biologique et de FXIII remis en solution; avant lyophilisation.

14. Procédé selon l'une quelconque des revendications 10 à 13, **caractérisé en ce que** le traitement thermique d'inactivation virale à sec est effectué sur les lyophilisats de fibrinogène, de colle biologique et de FXIII obtenus après l'étape de lyophilisation.

15. Procédé selon l'une quelconque des revendications 1 à 14, **caractérisé en ce qu'**il comprend une étape d'ultrafiltration de concentration effectuée préalablement à l'étape de diafiltration ou postérieurement à ladite étape, avant lyophilisation.

16. Procédé selon l'une quelconque des revendications 1 à 15, **caractérisé en ce qu'**il comprend, avant l'étape a), une étape initiale de prépurification de la fraction plasmatique solubilisée par un traitement à l'hydroxyde d'aluminium et/ou une précipitation à basse température.

17. Concentré lyophilisé de fibrinogène, susceptible d'être obtenu par la mise en oeuvre du procédé selon l'une quelconque des revendications 1 à 16, **caractérisé en ce qu'**il comprend le mélange des constituants du tampon de diafiltration défini à la revendication 8.

18. Concentré lyophilisé de colle biologique, susceptible d'être obtenu par la mise en oeuvre du procédé selon l'une quelconque des revendications 1 à 16, **caractérisé en ce qu'**il comprend le mélange des constituants du tampon de diafiltration défini à la revendication 8.

19. Concentré lyophilisé de Facteur XIII, susceptible d'être obtenu par la mise en oeuvre du procédé selon l'une quelconque des revendications 1 à 16, **caractérisé en ce qu'**il comprend le mélange des constituants du tampon de diafiltration défini à la revendication 8.

20. Concentré lyophilisé selon l'une des revendications 17 à 19, de qualité thérapeutique, obtenu par la mise en oeuvre du procédé selon l'une des revendications 10 à 14.
